# EUROPEAN PATENT SPECIFICATION

(11) **EP 0 788 564 B1**
(45) Date of publication and mention of the grant of the patent: **16.05.2001**
(21) Application number: 95934623.0
(22) Date of filing: 27.10.1995
(51) Int. Cl.: D06M 16/00, D06M 13/184

(54) **A PROCESS FOR CHEMICAL FINISHING OF INSOLUBLE POLYMER FIBRES**
VERFAHREN ZUR CHEMISCHEN AUSRÜSTUNG VON UNLÖSLICHEN POLYMERFASERN
PROCEDE D'APPRET CHIMIQUE DE FIBRES POLYMERES INSOLUBLES

(30) Priority: 28.10.1994 DK 125794
(43) Date of publication of application: 13.08.1997
(73) Proprietor: Novozymes A/S, 2880 Bagsvaerd (DK)
(72) Inventor: LUND, Henrik, DK-2880 Bagsvaerd (DK); KIRK, Ole, DK-2880 Bagsvaerd (DK); MUNK, Niels, DK-2880 Bagsvaerd (DK)
(86) International application number: PCT/DK95/00428
(87) International publication number: WO 96/13632

(56) References cited:
- DE-A- 4 012 351
- US-A- 5 042 986
- US-A- 5 191 071

## Description

The present invention relates to a process for chemical finishing of insoluble polymer fibres. More specifically, the invention relates to an enzymatic esterification of insoluble polymers wherein organic molecules containing a carboxylic acid residue are covalently linked to insoluble polymers such as cellulosic fibres or textile fabric, the fibre or fabric made therefrom having improved functional properties such as reduced tendency to wrinkling, improved durable softness, anti-static appearance, water repellency, enhanced soil release or flame retardancy.

### BACKGROUND OF THE INVENTION

In chemical finishing of textile fabrics two traditional ways of processing have been to either deposit a chemical compound or composition onto the fabric or to chemically reacting, e.g. cross-linking, certain useful compounds with the fabric.

Examples of deposit compounds are the application of softener finishes which may improve the hand and drape of fabrics, add body to the fabric, facilitate application of other finishes and increase the life and utility of the fabric. Softeners are usually available in three types: nonionic, anionic and cationic. As groups the anionic and nonionic softeners usually serve more as lubricants rather than softeners, while the cationic softeners are probably the best softening agents available. The cationic softeners impart a soft, silky and bulky hand in the fabric. The most common type of cationic softeners are the quaternary ammonium salts which have affinity for most textile fibers, as due to their positive charge. Since most fibers develop a negative surface charge in water, the cationic softeners exhaust onto the fibers.

In recent years there has, however, been an increasing enviromental concern about the usage of the quaternary ammonium salts as due to their poor bio-degradability as well as their fairly high toxicity.

Among reacting finishes for fabric, DMDHEU (Di-Methylol-Di-Hydroxy-Ethylene-Urea) is the most widely used for durable press treatment. At high temperature with presence of an acid catalyst it reacts with free hydroxy groups and creates internal crosslinks in the fibres. Release of residual formaldehyde compounds is a major concern, due to the toxicity of these compounds. An alternative formaldehyde free durable press treatment uses esterification with BTCA (1,2,3,4-Butane-Tetra-Carboxylic-Acid) at elevated temperatures in acid or with presence of a catalysts. The acid process causes damage to the fabric, whereas the catalysts e.g. hypophosphites have given reason to environmental concerns. These formaldehyde free treatments therefore have had limited application.

In order to obtain desirable end properties, paper or cardboard materials are conventionally finished with a synthetic coating such as oxidized polyethylenes, ethylene-acrylic acid, ketone resin, ureaformaldehyde, melamine formaldehyde, anionic latex etc. Due to environmental concerns, it is desirable to replace the petrochemically based coatings, with coatings based on renewable resources, in order to make entirely CO₂-neutral paper and cardboard materials.

Carboxylic ester hydrolases (EC 3.1.1), especially lipases and esterases, are well known as catalysts capable of catalyzing synthesis of esters by reacting an alcohol with either a carboxylic acid or an ester thereof solubilized in an organic solvent with low water-activity. Furthermore, these catalysts have proven efficient for esterification of various carbohydrates solubilized in organic solvents. Even though suspension of free enzymes have shown some effect immobilized enzymes have preferably been used as they exhibit improved stability in the organic solvents and, furthermore, offer obvious benefits regarding re-use of the catalyst. One example is the esterification of simple alkyl glucosides catalyzed by immobilized lipases in organic solvents with low water activity described in US-A-5,191,071.

Recently, various methods have been developed for solubilizing enzyme catalysts in organic media by either chemical modification of the enzyme with organic polymers such as polyethyleneglycol (PEG) (K. Takahashi et al. Biochem. Biophys. Re. Commun. 1984, 121, 261 and T. Yoshimoto et al. Biotech Lett. 1984, 6, 337 and K. Takahashi et al. J. Org. Chem. 1985, 50, 3414) or by complexing the catalyst with organic polymers (Y. Okahata and K. Ijiro, Bull. Chem. Jpn, 1992, 65, 2411). These methods have enabled reaction between solubilized organic substrates and soluble enzyme catalysts.

As described above, both immobilized and solubilized enzymes have been shown to be efficient for catalyzing esterification reactions by combining various solubilized substrates (including simple carbohydrates) in a suitable organic solvent with low water activity. However, no esterification of insoluble substrates has, so far, been described in the prior art.

### SUMMARY OF THE INVENTION

Surprisingly, it has been found that hydrolytic enzymes such as carboxylic ester hydrolases (EC 3.1.1), preferably lipases or esterases, are capable of catalyzing esterification of insoluble polymer substrates, preferably polymers such as cellulose and lignocellulose.

Based on this finding it is now possible to carry out chemical finishing of textile, fabric, yarn etc. containing insoluble polymer fibres, especially cellulosic or lignocellulosic fibres, by an enzymatic process in which a hydrolytic enzyme such as a lipase or an esterase catalyzes the formation of ester bonds between the polymer and a suitable reactant, i.e. an acyldonor preferably containing one or more carboxy functions.

Accordingly, the present invention provides a process for enzymatically catalyzing an esterification of insoluble polymer fibres containing free hydroxy groups, wherein the polymer fibre is reacted with a carboxylic acid or an ester thereof in the presence of an enzyme capable of catalyzing esterfication.

An advantage of the enzymatic esterification according to the invention is that the surfaces of porous materials can be modified selectively, since access to the interior of the material is restricted for the large enzyme molecules. Unlike many conventional processes, it may thus be possible to retain the basic structure of e.g. cellulosic fibres which have inherent strength and good chemical resistance properties. Further, it is comtemplated that surface esterification of fabric can be used to prepare hitherto unknown fabric types with improved fuctional properties. For example, it is contemplated that lipase catalyzed ester formation with fatty acids is useful for providing permanent wash-resistant water-repellancy to cotton or water-repellancy to cardboard boxes or containers, based on renewable materials.

Chemical finishing of textiles, fibres and yarns serves to improve the properties of the resulting product, usually a textile e.g. for garments, carpeting, upholstery. Examples of such properties are permanent press, softening, soil release, water repellancy and flame retardancy. The present invention provides a process by which, in dependance of the chemical compound actually attached to the polymer fibre by an ester bond, one or more of the desired properties may be obtained or improved in an easy, economical and environmentallyfriendly way.

Further, the process of the invention provides a durable finishing of the polymer fibre, i.e. provides a permanent improvement to the polymer fibre, which is in contrast to deposition finishings wherein a chemical compound is deposited on the polymer and, thus, may be easily removed mechanically when laundering or wearing or otherwise using the polymeric, preferably cellulosic or lignocellulosic, material.

In another aspect, the process of the invention relates to finishing of any insoluble polymeric material posessing free hydroxy groups, i.e. insoluble polymeric material present in other forms than as a fibre.

### DETAILED DESCRIPTION OF THE INVENTION

### The polymeric substrate

In the present specification and claims, the term "insoluble polymer fibre containing free hydroxy groups" is intended to mean a polymeric fibrous material having hydroxy groups capable of forming ester bonds when contacted with carboxy groups, i.e. carboxylic acids or esters thereof.

Preferably, the polymeric fibrous material subjected to the process of the invention is present as a fiber, a staple fiber such as a solvent-spun fiber, a filament, a thread, a yarn, or a textile fabric which may be woven, nonwoven or knitted.

In a preferred embodiment of the invention, the polymer fibre is a cellulosic polymer fibre, i.e. containing cellulose or cellulose derivatives, preferably prepared from cotton, viscose (rayon), lyocell, flax (linen), ramie, or any blend thereof; and blends thereof with polyesters, wool, polyamides and (poly)acrylics. Typical examples of such blends are viscose/cotton, viscose/polyester, lyocell/polyester, lyocell/cotton, cotton/acrylic, cotton/polyester, cotton/polyester/acrylic, cotton/polyamide/polyester.

In another preferred embodiment of the invention the polymer is a lignocellulosic fibre, for example paper or cardboard made from chemical or mechanical pulps of plant or wood fibres.

In yet another preferred embodiment of the invention, the polymer is synthetic polymer. Preferably, the synthetic polymer is selected from the group consisting of polyesters, polyamides, (poly)acrylics and co-polymers thereof.

### The carboxylic acid ester

In the present specification and claims, the term "carboxylic acid or an ester thereof" is intended to mean any carboxylic acid or ester which improves one or more properties of the polymeric material and is capable of forming ester bonds with the free hydroxy groups of the polymer.

It is contemplated that, e.g. for obtaining an improvement of the softening finish, i.e. improvement of the hand and drape of the final fabric, for obtaining flame retardancy, for obtaining water repellancy and for obtaining resin finishing ("permanent press"), it may be convenient to use in the process of this invention a carboxylic acid or an ester thereof with the general formula (I)

R-COOR¹ (I)

wherein
R is linear or branched C₁₋₂₅ alkyl, preferably linear C₃₋₂₅ alkyl, more preferably linear C₈₋₂₅ alkyl, which optionally is substituted with one or more carboxy, nitro, chloro, bromo, fluoro, amino, hydroxy, keto; and
R¹ is hydrogen, C₁₋₄ alkyl, or vinyl, preferably methyl, ethyl or vinyl.

For obtaining permanent press, it is advantageous to use a poly-carboxylic acid or an ester thereof, i.e. a carboxylic acid with two or more carboxy groups capable of forming ester bonds.

For obtaining flame retardancy, it is advantageous to use a halogen-substituted carboxylic acid or an ester thereof, i.e. a fluorinated, chlorinated or bromated carboxylic acid or an ester thereof.

Further, it is contemplated that the process of the invention is useful for dyeing, preferably for dyeing with a reactive textile dyestuff, the polymeric fibre or material by reacting the polymeric fibre or material with a carboxylic acid or ester thereof of the general formula (I)

R-COOR¹ (I)

wherein R comprises a chromophore and R¹ has the meaning set forth above. Usually, the chromophore comprises one or more heterocycles, preferably comprising one or more nitrogen, sulphur or oxygen atoms. Examples of useful chromophores are derivatives of acridines and phenazines.

Further, it is contemplated that the process of the invention is useful for obtaining brightness, e.g. optical brightness, of the polymeric material by reacting the polymeric material with an carboxylic acid or ester thereof of the general formula (I)

R-COOR¹ (I)

wherein R comprises a fluorophore and R¹ has the meaning set forth above. Examples of useful fluorophores are derivatives of xanthenes.

Further, it is contemplated that the process of the invention is useful for obtaining water repellancy of the polymeric material by reacting the polymeric material with a wax or a derivative of a wax containing one or more carboxy groups capable of forming ester bonds.

Other carboxylic acid esters which may advantageously be used in the process of the present invention are enolesters. By using enolesters in the process, the chemical reaction process is irreversible and results in a ketone or aldehyde reaction side product.

### The enzyme

In the present specification and claims, the terms "lipase" and "esterase" are intended to mean carboxylic ester hydrolases (EC 3.1.1), especially an enzyme that in an aqueous environment hydrolyses ester linkages present in either water-soluble molecules or water insoluble molecules (e.g. long chain lipids).

Enzymes suitable for the process of the present invention can be selected by the following method, utilizing the reversible nature of the esterification reaction, implicating that an enzyme able to catalyze ester formation will also be able to catalyze the hydrolysis of ester-bonds.

Cellulose esters are prepared according to the acid-chloride pyridine procedure of C.J. Malm et.al, (Ind. and Eng. Chem, Vol. 43, No.3, march 1951). The cellulose esters are incubated with the enzyme preparation in an agar gel containing phenol-red. 1000 ml agar gel is prepared from 17 g Agarose type 2 medium EEO, Sigma, A-6877, 3 gram NaNO₃, 1 gram K₂HPO₄, 0.5 gram KCl, 1 ml 1% FeSO₄ and 50 ml 0.4 gram/litre phenol-red solution.

If the enzyme hydrolyses the ester, carboxylic acid is liberated and will diffuse into the gel, changing the color from red to yellow. A number of controls must be run to avoid false positive or negative results: Ester without enzyme and enzyme without ester must both turn out negative in the test, and enzyme with glycerine tributyrate should react positively and give colour change.

The lipase is suitably a microbial or an animal-derived lipase. As such, the parent lipase may be selected from yeast, e.g. Candida lipases, bacterial, e.g. Pseudomonas lipases or fungal, e.g. Humicola or Rhizomucor lipases. More specifically, suitable lipases may be the *Rhizomucor miehei* lipase (e.g. prepared as described in EP-A-238 023), *Humicola lanuginosa* lipase e.g. prepared as described in EP-A-305 216 (available from Novo Nordisk under the trade name Lipolase™) , *Candida antarctica* lipase A or B, or *Pseudomonas cepacia* lipase. Other examples of suitable lipases are variants of any one of the lipases mentioned above, e.g. as described in WO-A-92/05249 or WO-A-93/11254. Examples of animal-derived lipases are lipases extracted or otherwise derived from porcine pancreas or from guinea-pigs.

A useful esterase is suitably one of microbial origin. As such the esterase may be either fungal, bacterial or from yeast.

Other useful enzymes are chemically modified lipases or esterases which may be obtained by the coupling of a polyethyleneglycol (PEG) to amino acid residues in the lipase as described in K. Takahashi et al. 1984, T. Yoshimoto et al., and K. Takahashi et al. 1985; or by complexing the lipase with organic polymers as described in Y. Okahata and K. Ijiro.

Other useful enzymes are lipases or esterases which are modified as described in WO-A-95/09909, i.e. glucosaminated enzymes having a higher pI than the unmodified (parent) enzyme.

### Definition of Lipase Units (LU)

The lipase activity is determined in the following assay: A substrate for lipase is prepared by emulsifying glycerine tributyrat (MERCK) using gum-arabic as emulsifier. Lipase activity is assayed at pH 7 using pH stat method. One unit of lipase activity (LU) is defined as the amount of enzyme needed to liberate one micromole (1 *µ* M) fatty acid per minute.

### Process conditions

It is obvious to the skilled person that the process must be carried out under conditions (e.g. temperature, pH, solvent) which favours the esterification process over the undesired hydrolytic cleavage of ester bonds. Accordingly, it is impossible to carry out the desired esterification process when using water as a solvent.

The process of the invention may be carried out in a suitable solvent. Preferably, the solvent is a water-immiscible organic solvent. It is contemplated that conventional organic solvents except alcohols are useful in the process of the invention. Examples of useful solvents are iso-octane, n-hexane and cyclohexane.

However, when the carboxylic acid or the ester to be used is liquid at the process temperature, the process may be carried out without a solvent.

Alternatively, the reaction may take place in a microemulsion formed by adding an carboxylic acid or an ester thereof to a mixture of water and a suitable surfactant. Typically, the surfactant is a nonionic surfactant.

The following non-limiting example illustrates the invention.

### EXAMPLE 1

A cotton swatch (2 x 2 cm) was added to a solution of decanoic acid (50 mg) in butanone (10 ml). Lipase A from *Candida antarctica* (5 LU/mg, available from Novo Nordisk A/S) was then added and the mixture was vigorously stirred at 50°C for 24 hours. The swatch was then rinsed thoroughly in butanone (3 x 10 ml) and dried at room temperature for 2 hours. The swatch was then treated with an aqueous solution (5 ml) of sodium hydroxide (1 M) at 40°C for 1 hour. The swatch was removed and the solution was then acidified with hydrochloric acid to a pH of 2. Extraction of this solution with chloroform (5 ml) afforded an extract which was evaporated *in vacuo.* Analysis by NMR spectroscopy (employing a Bruker acp 300 NMR spectrometer and using deuterated chloroform as solvent) indicated the extract to contain decanoic acid.

Another swatch was treated as above, only adding no enzyme to the butanone. NMR analysis failed to prove any presence of decanoic acid in the final chloroform extract.

Accordingly, this example shows that the first swatch had been successfully esterified with decanoic acid in the presence of Lipase A as the catalyst.

### EXAMPLE 2

### Lipase-catalyzed surface modification of cotton

For preparation of a butyrate (C4) esterified fabric, an 8x8 cm swatch of pure cotton knit was dried, and immersed in a solution of 1 g vinyl-butyrate dissolved in 160 ml iso-octane. 200 micro litre of lipase derived from PEG modified *Candida antarctica* Lipase A (5 LU/mg) *was* mixed into the solution, and the solution with the swatch was incubated 4 hours at 60°C under gentle stirring. The swatch was removed, dried, washed with demineralized water and dried again.

Another swatch was surface derivatized into a laurate (C12) ester by similar treatment, but instead of 1 g vinyl-butyrate, 1.5 g vinyl-laurate was used. After incubation this swatch was washed with methanol, before washing with demineralized water and drying.

A third swatch was used as reference

The formation of the ester bonds was verified with photoacoustic Fourier transformed infrared spectroscopy, showing carbonyl ester peaks near 1740 cm⁻¹, except for the reference swatch.

### Test of water-absorptive properties

The water absorptive properties of the modified cotton swatches was tested with a Tensiometer (Sigma 70, KSV, Finland). With a constant rate of 20 mm/second this apparatus cyclically dips the swatch 2mm into water and elevates it to 5 mm above the water surface. It then dips the swatch again, and repeats the cycle. The weight of the swatches between each dip is recorded. The results are shown in table 1.

**TABLE 1**

| Water uptake g/g | Reference | C-4 cellulose ester | C12 cellulose ester |
|---|---|---|---|
| 0 dips | 0 | 0 | 0 |
| 2 dips | 0.8 | 2.6 | 0.3 |
| 4 dips | 1.3 | 2.7 | 0.7 |
| 6 dips | 1.6 | 2.7 | 0.9 |
| 8 dips | 1.9 | 2.7 | 1.1 |
| 10 dips | 2.0 | 2.7 | 1.2 |
| 12 dips | 2.3 | 2.7 | 1.3 |

The results show that the surface treatments have caused major changes in the properties of the cotton knit. The C-4 esterified cotton takes up water at a higher rate than the reference, whereas the C-12 esterified cotton has reduced the water-uptake two-fold.

A drop test was also carried out: Droplets of 10 *µ*-litre demineralized water was placed on the surface of each swatch. The time elapsed when the droplets had disappeared was recorded. Results from three repeated experiments are given in table 2.

**TABLE 2**

| | seconds | seconds | seconds |
|---|---|---|---|
| Reference | 20 | 20 | 20 |
| C-4 esterified | 1 | 1 | 2 |
| C-12 esterified | >3600 | >3600 | >3600 |

The results show that the C4-esterified cotton has much enhanced water suction performance compared to the reference, and thus is particularly suited for use in e.g. towels. The C-12 esterified cotton has become completely repellant to water droplets, and thus could be particularly useful in e.g. out-door wear.

### REFERENCES

K. Takahashi et al.: Biochem. Biophys. Re. Commun. 1984, 121, 261
T. Yoshimoto et al. Biotech Lett. 1984, 6, 337
K. Takahashi et al., J. Org. Chem. 1985, 50, 3414
Y. Okahata and K. Ijiro, Bull. Chem. Jpn, 1992, 65, 2411
C.J. Malm et.al, Ind. and Eng. Chem, Vol. 43, No.3, March 1951

## Claims

1. A process for enzymatically catalyzing an esterification of insoluble polymer fibres containing free hydroxy groups, wherein the polymer fibres are reacted with a carboxylic acid or an ester thereof in the presence of an enzyme capable of catalyzing esterfication.

2. The process according to claim 1, wherein the polymer fibres are present as yarn, textile, fabric, papermaking pulp, cardboard or paper.

3. The process according to claim 1 or 2, wherein the polymer is a cellulosic polymer, preferably a cellulosic polymer selected from the group consisting of cotton, viscose (rayon), lyocell, flax (linen), ramie, and any blend thereof; and blends thereof with polyesters, wool, polyamides and (poly)acrylics.

4. The process according to claim 1 or 2, wherein the polymer is a synthetic polymer, preferably a synthtic polymer selected from the group consisting of polyesters, polyamides, and (poly)acrylics.

5. The process according to claim 1 or 2, wherein the polymer fibres are lignocellulosic polymer fibres, preferably lignocellulosic polymer fibres obtained from wood or plants, more preferably paper or cardboard made from chemical or mechanical pulps of plant or wood fibres.

6. The process according to any of the claims 1-5, wherein the carboxylic acid or the ester thereof has the general formula (I)
R-COOR¹ (I)
wherein
R is linear or branched C₁₋₂₅ alkyl, preferably linear C₃₋₂₅ alkyl, more preferably linear C₈₋₂₅ alkyl, which optionally is substituted with one or more carboxy, nitro, chloro, bromo, fluoro, amino, hydroxy, keto; and
R¹ is hydrogen, C₁₋₄ alkyl, or vinyl, preferably methyl, ethyl or vinyl.

7. The process according to any of the claims 1-5, wherein the carboxylic acid or the ester thereof has the general formula (I)
R-COOR¹ (I)
wherein R comprises a chromophore or a fluorophore.

8. The process according to any of the claims 1-6, wherein the carboxylic acid or the ester thereof is a wax or is derived from a wax.

9. The process according to any of the claims 1-6, wherein the carboxylic acid ester is an enolester, preferably vinylester or isopropenylester.

10. The process according to any of the claims 1-9, wherein the enzyme is a lipase, preferably a microbial lipase, which optionally is chemically modified.

11. The process according to any of the claims 1-9, wherein the enzyme is an esterase.

## Patentansprüche

1. Verfahren zur enzymatischen Katalyse einer Veresterung von unlöslichen Polymerfasern, die freie Hydroxygruppen enthalten, wobei die Polymerfasem mit einer Carbonsäure oder einem Ester davon in Gegenwart eines Enzyms, welches zur Katalyse der Veresterung in der Lage ist, umgesetzt werden.

2. Verfahren nach Anspruch 1, worin die Polymerfasern als Garn, Textilie, Gewebe, Pulpe zur Papierherstellung, Karton oder Papier vorliegen.

3. Verfahren nach Anspruch 1 oder 2, worin das Polymer ein Cellulosepolymer ist, vorzugsweise ein Cellulosepolymer, welches aus der Gruppe, bestehend aus Baumwolle, Viskose (Kunstseide), Lyocell, Flachs (Leinen), Ramie und irgendeiner Mischung davon, sowie Mischungen davon mit Polyestern, Wolle, Polyamiden und (Poly)acrylmaterialien, ausgewählt ist.

4. Verfahren nach Anspruch 1 oder 2, worin das Polymer ein synthetisches Polymer ist, vorzugsweise ein synthetisches Polymer, welches aus der Gruppe, bestehend aus Polyestern, Polyamiden und (Poly)acrylmaterialien, ausgewählt ist.

5. Verfahren nach Anspruch 1 oder 2, worin die Polymerfasern Lignocellulose-Polymerfasern, vorzugsweise aus Holz oder Pflanzen erhaltene Lignocellulose-Polymerfasern, bevorzugter Papier oder Karton, hergestellt aus chemischen oder mechanischen Pulpen von Pflanzen- oder Holzfasern, sind.

6. Verfahren nach irgendeinem der Ansprüche 1-5, worin die Carbonsäure oder deren Ester die allgemeine Formel (I)
R-COOR¹ (I)
aufweist, worin
R ein lineares oder verzweigtes C₁₋₂₅-Alkyl, vorzugsweise ein lineares C₃₋₂₅-Alkyl, bevorzugter ein lineares C₈₋₂₅-Alkyl, ist, welches gegebenenfalls mit einem oder mehreren Carboxy, Nitro, Chlor, Brom, Fluor, Amino, Hydroxy, Keto substituiert ist; und
R¹ Wasserstoff, C₁₋₄-Alkyl oder Vinyl, vorzugsweise Methyl, Ethyl oder Vinyl, ist.

7. Verfahren nach irgendeinem der Ansprüche 1-5, worin die Carbonsäure oder deren Ester die allgemeine Formel (I)
R-COOR¹ (I)
aufweist, worin R ein Chromophor oder ein Fluorophor umfaßt.

8. Verfahren nach irgendeinem der Ansprüche 1-6, worin die Carbonsäure oder deren Ester ein Wachs ist oder von einem Wachs abgeleitet ist.

9. Verfahren nach irgendeinem der Ansprüche 1-6, worin der Carbonsäureester ein Enolester, vorzugsweise ein Vinylester oder Isopropenylester, ist.

10. Verfahren nach irgendeinem der Ansprüche 1-9, worin das Enzym eine Lipase ist, vorzugsweise eine mikrobielle Lipase, welche gegebenenfalls chemisch modifiziert ist.

11. Verfahren nach irgendeinem der Ansprüche 1-9, worin das Enzym eine Esterase ist.

## Revendications

1. Un procédé pour catalyser de façon enzymatique une estérification de fibres polymères insolubles renfermant des groupes hydroxy libres, dans lequel les fibres polymères sont amenées à réagir avec un acide carboxylique ou un ester de celui-ci, en présence d'une enzyme capable de catalyser l'estérification.

2. Le procédé selon la revendication 1, dans lequel les fibres polymères sont présentes sous forme de brins, de textile, de tissu, de pâte de fabrication de papier, de carton ou de papier.

3. Le procédé selon la revendication 1 ou 2, dans lequel le polymère est un polymère cellulosique, de préférence, un polymère cellulosique choisi parmi le groupe comprenant le coton, la viscose (rayonne), le lyocell, le lin, la ramie et n'importe quel mélange de ceux-ci, et leurs mélanges avec des polyesters, de la laine, des polyamides et des (poly)acryliques.

4. Le procédé selon la revendication 1 ou 2, dans lequel le polymère est un polymère synthétique, de préférence, un polymère synthétique choisi parmi le groupe comprenant les polyesters, les polyamides et les (poly)acryliques.

5. Le procédé selon la revendication 1 ou 2, dans lequel les fibres polymères sont des fibres polymères lignocellulosiques, de préférence, des fibres polymères lignocellulosiques obtenues à partir de bois ou de plantes, de préférence de papier ou de carton obtenu à partir de pâtes chimiques ou mécaniques de fibres de bois ou de plantes.

6. Le procédé selon l'une quelconque des revendications 1 à 5, dans lequel l'acide carboxylique ou son ester répond à la formule générale (I)
R-COOR¹ (I)
dans laquelle
R est un radical alkyle linéaire ou ramifié en C₁₋₂₅, de préférence un radical alkyle linéaire en C₃₋₂₅, mieux encore un radical alkyle linéaire en C₈₋₂₅ qui est éventuellement substitué avec un ou plusieurs groupes carboxy, nitro, chloro, bromo, fluoro, amino, hydroxy, céto ; et
R¹ est l'hydrogène, un radical alkyle en C₁₋₄ ou un radical vinyle, de préférence un radical méthyle, éthyle ou vinyle.

7. Le procédé selon l'une quelconque des revendications 1 à 5, dans lequel l'acide carboxylique ou son ester répond à la formule générale (I)
R-COOR¹ (I)
dans laquelle R renferme un chromophore ou un fluorophore.

8. Le procédé selon l'une quelconque des revendications 1 à 6, dans lequel l'acide carboxylique ou son ester est une cire ou est dérivé d'une cire.

9. Le procédé selon l'une quelconque des revendications 1 à 6, dans lequel l'ester d'acide carboxylique est un énolester, de préférence un ester de vinyle ou un ester d'isopropényle.

10. Le procédé selon l'une quelconque des revendications 1 à 9, dans lequel l'enzyme est une lipase, de préférence une lipase microbienne qui est éventuellement modifiée chimiquement.

11. Le procédé selon l'une quelconque des revendications 1 à 9, dans lequel l'enzyme est une estérase.
